## Europäisches Patentamt
## European Patent Office
## Office européen des brevets

(19)

(11) Veröffentlichungsnummer: **0 186 134**
**B1**

(12) # EUROPÄISCHE PATENTSCHRIFT

(45) Veröffentlichungstag der Patentschrift:
15.02.89

(51) Int. Cl.⁴: **C 12 Q 1/26**, C 12 Q 1/28, G 01 N 33/52

(21) Anmeldenummer: 85116213.1

(22) Anmeldetag: 19.12.85

(54) **Mittel zur Verbesserung des Nachweises Wasserstoffperoxid liefernder Oxidase-Reaktionen und seine Verwendung.**

(30) Priorität: 20.12.84 DE 3446637

(43) Veröffentlichungstag der Anmeldung:
02.07.86 Patentblatt 86/27

(45) Bekanntmachung des Hinweises auf die Patenterteilung:
15.02.89 Patentblatt 89/7

(84) Benannte Vertragsstaaten:
AT BE CH DE FR GB IT LI LU NL SE

(56) Entgegenhaltungen:
EP-A-0 054 358
EP-A-0 071 730
EP-A-0 100 217

CHEMICAL ABSTRACTS, Band 102, Nr. 15, 15. April 1985, Seite 273, no. 127903b, Columbus, Ohio, US; & JP - A - 59 66 899 (ONO PHARMACEUTICAL CO. LTD., TOYOBO CO. LTD.) 16.04.1984
Römpps Chemie-Lexikon, Seite 3406

(73) Patentinhaber: BOEHRINGER MANNHEIM GMBH, Sandhofer Strasse 112- 132, D-6800 Mannheim-Waldhof (DE)

(72) Erfinder: Siedel, Joachim, Dr., Bahnhofstrasse 6, D-8139 Bernried (DE)
Erfinder: Röder, Albert, Dr., Tiefentalweg 8, D-8124 Seeshaupt (DE)
Erfinder: Ziegenhorn, Joachim, Dr., Ina- Seidel-Weg 1, D-8130 Starnberg (DE)

(74) Vertreter: Weickmann, Heinrich, Dipl.- Ing., Patentanwälte Dipl.- Ing. H.Weickmann Dipl.-Phys.Dr. K.Fincke Dipl.- Ing. F.A.Weickmann Dipl.- Chem. B. Huber Dr.- Ing. H. Liska Dipl.-Phys.Dr. J. Prechtel Postfach 860820, D-8000 München 86 (DE)

**Beschreibung**

Durch die Entwicklung einfacher und genauer Nachweisverfahren für $H_2O_2$ in wäßrigem, neutralem Medium hat in den vergangenen Jahren die Verwendung $H_2O_2$-liefernder Oxidase-Reaktionen erhebliche Bedeutung in der enzymatischen Analyse erlangt. Dies gilt insbesondere für die Routinebestimmung verschiedener in Körperflüssigkeiten wie Blutserum bzw. -plasma oder Urin enthaltenen Substanzen mit hohem klinischdiagnostischen Stellenwert.

Zur quantitativen Bestimmung von $H_2O_2$, welches entweder direkt oder indirekt durch Vorschaltung geeigneter, vorzugsweise enzymatischer Umwandlungsreaktionen aus der letztlich zu bestimmenden Substanz mit Hilfe derartiger, spezifischer Oxidase-Reaktionen gebildet wird, werden neben elektrochemischen Meßverfahren bevorzugt colorimetrische Nachweismethoden eingesetzt. Als besonders brauchbar haben sich dabei solche vom "Trinder-Typ" erwiesen, bei denen in Gegenwart von Peroxidase, E.C. 1.11.1.7, aus Phenol bzw. Phenolderivaten oder substituierten Anilinen und 4-Aminoantipyrin ein roter bis violetter Farbstoff gebildet wird, dessen Menge bzw. Intensität in linearem Verhältnis zur umgesetzten $H_2O_2$-Menge steht.

Daneben haben auch noch auf einem ähnlichen Prinzip beruhende Farbindikatorsysteme eine gewisse Bedeutung erlangt, bei denen statt 4-Aminoantipyrin 3-Methyl-2-benzothiazolinon-Hydrazon ("MBTH") bzw. das in 2'-Stellung sulfonierte Analogon ("MBTH-S") als Kuppler eingesetzt wird, und zwar vorzugsweise in Kombination mit substituierten Anilinen (siehe z. B. Clin. Chem. 17, 1154 - 1159 (1971)). Es hat sich jedoch gezeigt, daß diese Systeme bevorzugt nur in Reagenzien verwendet werden können, deren pH-Wert ⩽ 7 beträgt; andernfalls kann es zu einer autoxidativen Farbbildung bereits in Abwesenheit von $H_2O_2$ kommen, die zu einer relativ raschen Einfärbung und damit einer deutlich begrenzten Lagerfähigkeit des gebrauchsfertigen Reagenzes führt.

Die Vorteile derartiger Analysenmethoden z. B. gegenüber auf Dehydrogenase-Reaktionen basierenden Testverfahren, bei denen die Bildung oder der Verbrauch von NAD(P)H im UV-Bereich gemessen wird, liegen neben der meist deutlich längeren Haltbarkeit des Analysenreagenzes und den wesentlich geringeren, zum Teil sogar vernachlässigbaren Interferenzen des Testansatzes durch Probenmaterial-Eigenfärbungen oder -Trübungen im sichtbaren Wellenlängenbereich insbesondere in einer gewissen Flexibilität (Steuerbarkeit, Beeinflußbarkeit) hinsichtlich der Empfindlichkeit der Nachweisreaktion, je nachdem, welche der prinzipiell für die angewendete Farbreaktion geeigneten chromogenen Verbindungen eingesetzt werden.

Allerdings sind dieser Flexibilität nach oben hin Grenzen gesetzt: Zum Beispiel kann ohne weiteres mit dem Farbindikatorsystem Peroxidase/Phenol/4-Aminoantipyrin, dessen Nachweisempfindlichkeit etwa der einer NAD(P)+-abhängigen Dehydrogenase-Reaktion vergleichbar ist (vgl. "Methods in Enzymatie Analysis", 3. Ausgabe (Verlag Chemie, Weinheim 1983), Bd. I, Kap. 2.6.1.2), ein relativ hochkonzentrierter Serumbestandteil wie Gesamtcholesterin (Summe aus freiem und Fettsäure-verestertem Cholesterin, Normalkonzentration ca. 5,7 mmol/l; Nachweis über die Kopplung der von Cholesterin-Esterase, E.C. 3.1.1.13, sowie Cholesterin-Oxidase, E.C. 1.1.3.6 katalysierten Reaktionen, die letztlich $H_2O_2$ bilden) selbst bei einem Proben-Einsatz von nur 20 µl auf 2,0 ml Analysenreagenz mit hoher Genauigkeit (Meßsignal im normalen Konzentrationsbereich ca. 0,26 bzw. 0,38 Extinktionseinheiten bei 546 nm bzw. $\lambda_{max}$ = 500 nm) und Vernachlässigung der Probeneigenextinktion bestimmt werden.

Im Gegensatz dazu ist z. B. im Falle der Serum-Creatinin-Bestimmung (Nachweis über die Hintereinanderschaltung der von Creatininase, E.C. 3.5.2.10, Creatinase, E.C. 3.5.3.3, sowie Sarcosin-Oxidase, E.C. 1.5.3.1, katalysierten Reaktionen) (pH-Optimum ca. 8,0) selbst bei Verwendung des zur Zeit empfindlichsten bekannten Trinder-Farbindikatorsystems (2,4,6-Trijod- bzw. 2,4,6-Tribrom-3-hydroxybenzoesäure als Farbkuppler mit 4-Aminoantipyrin; ca. 5-fach höhere $H_2O_2$-Nachweisempfindlichkeit gegenüber Phenol als Kuppler, s. Clin. Chem. 26, 1062 (1980) bzw. Ann. Clin. Biochem. 21, 430 - 433 (1984)) sowie einer so hohen Dosierung wie 100 µl Probe auf 2,0 ml Analysenreagenz das Meßsignal pro 1 mg/dl Creatinin-Probenkonzentration (Obergrenze der Normalkonzentration im Serum ca. 88 µmol/l = 1 mg/dl) mit ca. 0,07 bzw. 0,1 Extinktionseinheiten bei 546 nm bzw. $\lambda_{max}$ = 510 nm noch nicht ausreichend, um die Creatinin-Bestimmung mit einer dem Cholesterin-Test vergleichbaren, hinreichenden Präzision, d. h. einem Variationskoeffizienten von ca. 2 % im Entscheidungsbereich, durchführen zu können.

Im Prinzip könnte dieses Ziel zwar durch eine Erhöhung des Probe-/Reagenz-Volumenverhältnisses erreicht werden, jedoch ist bei einer solchen Maßnahme erfahrungsgemäß mit deutlich ins Gewicht fallenden Störungen der Messung u. a. bei der Analyse trüber (lipämischer, d.h. triglyceridreicher) Proben oder durch andere im Probenmaterial potentiell enthaltene, die Farbreaktion negativ beeinflussende, reduzierende Bestandteile wie Ascorbinsäure, Bilirubin sowie bestimmte Pharmaka zu rechnen.

Ähnliches gilt auch für andere, niedrig konzentrierte Serum-Metabolite wie z. B. Harnsäure (Normalwert im Serum 140 bis 400 µmol/l). Im Falle der Serum-Harnsäure-Bestimmung (mittels Uricase, E.C. 1.7.3.3) werden üblicherweise nur maximal 50 µl Probe pro 2,0 ml Analysenreagenz eingesetzt, um auf die Mitführung eines Probenleerwerts (was bei der Serum-Creatinin-Bestimmung wegen des gleichzeitig in signifikanten Mengen vorhandenen Creatins unumgänglich ist) verzichten zu können und damit die Durchführung der Analyse zu erleichtern. Unter diesen Bedingungen werden mit dem Trijod- bzw. Tribromhydroxybenzoesäure/4-Aminoantipyrin-Farbindikatorsystem im Entscheidungsbereich ebenfalls nur relativ niedrige Extinktionswerte erhalten (ca. 0,045 bis 0,13 bei 546 nm bzw. 0,064 bis 0,186 bei 510 nm).

Schließlich wurde z. B. bei der Creatinin-Bestimmung nach dem obengenannten Reaktionsschema mit Farbindikatorsystemen vom Trinder-Typ (Tribrom- bzw. Trijodhydroxybenzoesäure als phenolischer oder N-

**EP 0 186 134 B1**

Ethyl-N-β-sulfoethyl-m-toluidin, "EST", als anilinischer Kupplungspartner für 4-Aminoantipyrin) auch gefunden, daß bei Verwendung wäßriger Creatinin-Standards verschiedener Konzentrationen die resultierende Bezugsgerade nicht durch den Ursprung des Koordinatensystems verläuft (x-Achse: Konzentration von Creatinin in der Probe; y-Achse: Farbmeßsignal in Extinktionseinheiten), sondern einen negativen y-Achsenabschnitt in der Größenordnung von ca. 0,015 (Tribromhydroxybenzoesäure/4-Aminoantipyrin-System, $\lambda$ = 546 nm) oder ca. 0,013 (EST/4-Aminoantipyrin-System, $\lambda$ = 578 nm) aufweist, was immerhin etwa 20 bis 33 % des Normalwertsignals ausmacht (siehe Beispiele 1 und 4) und die Kalibrierung solcher Tests insbesondere bei Einpunkt-Eichung erheblich verfälscht.

Aus CA, Band 102, Nr. 15, Seite 273, Referat 127903b war ein Verfahren zur Bestimmung von Guanase bekannt, bei dem von Xanthinoxidase gebildetes $H_2O_2$ bestimmt wird. Da Xanthinoxidase bei einem pH von 7 bereits 20 % und bei pH 8 sogar 80 % $O_2^-$-Radikal bildet, wird bei diesem Verfahren Superoxiddismutase zugesetzt, um gebildetes $O_2^-$ zu $H_2O_2$ und $O_2$ umzusetzen und so eine maximale Farbausbeute bei dem $H_2O_2$-Farbindikatorschritt zu erzielen. Bei Kenntnis dieser Entgegenhaltung schien der Zusatz einer Superoxiddismutase nur sinnvoll bei Reaktionen, bei denen $O_2^-$ produzierende Oxidasen verwendet werden.

Aufgabe der Erfindung ist daher ein Verfahren bzw. Mittel zu schaffen, welches einerseits die Empfindlichkeit des Nachweises $H_2O_2$-liefernder Oxidase-Reaktionen als solche deutlich zu steigern vermag und andererseits auch das Auftreten von Achsenabschnitten bei der Erstellung zugehöriger Eichgeraden verhindert, den Nachweis $H_2O_2$-liefernder Oxidase-Reaktionen also insgesamt wesentlich verbessert.

Gelöst wird diese Aufgabe erfindungsgemäß durch ein Verfahren zur verbesserten colorimetrischen Bestimmung von $H_2O_2$, welches durch eine $H_2O_2$-liefernde Oxidase gebildet wird, durch Zusatz eines chromogenen Systems und Messung des gebildeten Farbstoffs, welches dadurch gekennzeichnet ist, daß man der Reagenzlösung Superoxiddismutase E.C. 1.15.1.1 zusetzt.

Als besonders geeignet erwies sich im Rahmen der Erfindung die Cu, Zn-Superoxiddismutase. Dieses Enzym läßt sich aus Erythrocyten gewinnen, beispielsweise solchen aus Rinderblut. Es weist je 2 Mol Kupfer und Zink und ein Molekulargewicht um etwa 33.000 D auf. Cu-, Zn-Superoxiddismutase findet sich aber auch in anderen Materialien, z. B. Hefe.

Die Superoxiddismutase wird vorzugsweise in einer Menge von 10 bis 150 µg/ml Reagenzlösung, besonders bevorzugt in einer Menge von 25 bis 75 µg/ml Lösung zugesetzt. 1 µg Superoxiddismutase entspricht hierbei etwa 4 U der handelsüblichen Superoxiddismutase-Präparate. Diese Relation beruht auf der in J. Biol. Chem. 244, 6049 (1969) beschriebenen Methode zur Bestimmung der Aktivität der Superoxiddismutase.

Als chromogene Systeme können im Rahmen der Erfindung prinzipiell alle mit $H_2O_2$ unter Farbstoffbildung reagierenden chromogenen Systeme verwendet werden, solange sie keine Bestandteile enthalten, welche die Aktivität der Superoxiddismutase oder eventuell vorhandener anderer Enzyme beeinträchtigen. Zahlreiche derartige chromogene Systeme sind dem Fachmann bekannt und bedürfen hier keiner näheren Erläuterung. Bevorzugt wird ein chromogenes System vom Trindertyp. Dieses enthält als wesentliche Bestandteile Peroxidase, Phenol oder ein Phenol- oder Anilinderivat und 4-Aminoantipyrin. Besonders bevorzugte Phenolderivate hierfür sind 2,4,6-Trijod-3-hydroxybenzoesäure oder die entsprechende Tribromverbindung. Bevorzugte Anilinderivate sind N-Ethyl-N-β-sulfoethyl-m-toluidin oder N-Ethyl-N-β-hydroxyethyl-m-toluidin. Ein weiteres bevorzugtes chromogenes System besteht aus Peroxidase, einem Anilinderivat und MBTH (3-Methyl-2-benzothiazolinon-hydrazon) bzw. MBTH-S. In diesem System werden als Anilinderivate, außer den oben bereits genannten beiden Verbindungen, auch N,N-Dimethylanilin und N,N-Dimethylaminobenzoesäure bevorzugt eingesetzt.

Besonders gute Ergebnisse zeigt die Erfindung bei den $H_2O_2$-liefernden Oxidasen Sarcosinoxidase, Uricase und Oxalatoxidase. Aber auch andere $H_2O_2$-liefernde Oxidasen können verwendet werden.

Dieser Befund war nicht vorhersehbar und läßt sich derzeit auch nicht befriedigend erklären, da er sich nicht mit der bekannten enzymatischen Eigenschaft der Superoxiddismutase, nämlich der Katalyse der Reaktion 2 $O_2^- + 2 H^+ \rightarrow H_2O_2 + {}^+O_2$ in Einklang bringen läßt:

Zum einen ist weder von Oxidasen wie der Sarcosin-Oxidase oder der Uricase bekannt, daß sie während der Umsetzung ihres jeweiligen Substrats, Sarcosin bzw. Harnsäure, neben $H_2O_2$ noch in signifikantem Umfang $O_2^-$-Radikale liefern, was entsprechend der oben aufgeführten Reaktionsgleichung eine zusätzliche $H_2O_2$-Bildung und damit eine Verbesserung der $H_2O_2$-Nachweisreaktion durch den Superoxiddismutase-Zusatz hätte erklären können; z. B. wird in Agric. Biol. Chem. 44, 1391 (1980) ausdrücklich darauf hingewiesen, daß die Sarcosin-Oxidase aus Cylindrocarpum didymum M-1 bei der Sarcosin-Oxidation in stöchiometrischen Mengen $H_2O_2$ liefert. Eine gleiche Feststellung wurde für die Sarcosin-Oxidase aus Corynebacterium getroffen (J. Biochem. 89, 599 (1981)). Auch bei der Harnsäure-Oxidation mittels Uricase wurde keinerlei Bildung des Superoxid-Radikals neben der $H_2O_2$-Bildung nachgewiesen (Arch. Biochem. Biophys. 163, 359 (1974)).

Zum anderen spricht gegen eine Wirkung der Superoxiddismutase gemäß der oben aufgeführten Reaktionsgleichung auch der Befund, daß bei Ersatz der Superoxiddismutase durch Kupferkomplexe, die eine ausgeprägte, zum Teil die Superoxiddismutase sogar übertreffende Wirksamkeit zur $O_2^-$-Disproportionierung aufweisen, wie Cu(acetylsalicylat)$_2$, Cu(lysin)$_2$ oder Cu$_2$(indomethacin)$_4$ (vgl. Bull. europ. Physiopath. resp. 17 (suppl.), 73 (1981)), selbst bei Konzentrationen im Oxidase-Reagenz bis zu 100 µmol/l (die höchste verwendete Superoxiddismutase-Konzentration in den nachfolgenden Beispielen beträgt nur 3,8 µmol/l!) keinerlei Verbesserung des $H_2O_2$-Nachweises bewirkt wird.

Weiterhin ist die Wirkung der Superoxiddismutase auch in Bezug auf die Achsenabschnitts-Beseitigung nicht anhand des obengenannten Reaktionsschemas erklärbar, da - selbst wenn eine gewisse $O_2^-$-Bildung

3

seitens der erwähnten Oxidasen, entgegen den Literaturangaben, doch vorläge - diese bei gegen Null gehendem Substratumsatz ebenfalls gegen Null gehen müßte.

Schließlich ist der den Nachweis $H_2O_2$-liefernder Oxidase-Reaktionen verbessernde Effekt des Superoxiddismutase-Zusatzes gleichermaßen ausgeprägt in Phosphatfreiem Reagenz (Pufferung z. B. durch TES, 100 mmol/l) als auch in mit Phosphat (Konzentration im Reagenz 100 bis 150 mmol/l) gepufferter Lösungen, obwohl für die besonders bevorzugte Superoxiddismutase aus Rindererythrozyten in der Literatur beschrieben ist, daß ihre enzymatische Aktivität durch Phosphat stark gehemmt wird, und zwar bereits zu ca. 50 % bei einer Phosphat-Konzentration von nur 10 mmol/l (Biochemistry 23, 2079 (1984)).

Ein weiterer Gegenstand der Erfindung ist ein Reagenz zur verbesserten colorimetrischen Bestimmung von $H_2O_2$, enthaltend eine $H_2O_2$-liefernde Oxidase, ein chromogenes System, Puffer und gegebenenfalls Hilfsenzyme, welches dadurch gekennzeichnet ist, daß es Superoxiddismutase enthält.

Für die Zusammensetzung des Reagenz gelten im übrigen die oben in bezug auf das Verfahren gemachten Ausführungen sinngemäß. Für die Analyse von Serum-Bestandteilen ist es günstig, den erfindungsgemäßen Reagenzien noch ein Aufklarungssystem zuzufügen, z. B. in Form einer Mischung eines nichtionischen Tensids mit Salzen von Gallensäuren wie Na-Cholat sowie einer Lipase, E.C. 3.1.1.3, um auch lipämische Proben ohne Störung durch Hintergrundtrübungen messen zu können. Schließlich können die Reagenzien vorzugsweise auch noch ein Konservierungsmittel wie Na-Azid, Mittel zur Beseitigung von durch Ascorbinsäure- und Bilirubin-haltigen Proben hervorgerufenen Störungen der $H_2O_2$-Nachweisreaktion wie Ascorbat-Oxidase und Kaliumferrocyanid, sowie kleine Mengen von Schwermetall-Komplexbildnern wie EDTA enthalten. Die Mengenanteile bzw. Konzentrationen dieser Substanzen, der Bestandteile des chromogenen Systems und Puffer entsprechen denen der bekannten Reagenzien und bedürfen daher hier keiner weiteren Erläuterung.

Das erfindungsgemäße Reagenz kann auch auf Teststreifen imprägniert vorliegen und sog. "trockenchemische" Analysen erlauben.

Durch den erfindungsgemäßen Zusatz läßt sich die Empfindlichkeit des $H_2O_2$-Nachweises um ca. 22 bis 34 % steigern, besonders bei den bevorzugten farbgebenden Systemen vom Trinder-Typ, (je nach Art der verwendeten Oxidase-Reaktion sowie der Zusammensetzung des zugehörigen Analysenreagenzes) und gleichzeitig auch das Auftreten von Achsenabschnitten bei der Erstellung von Eichgeraden (sowohl bei Indikatorsystemen vom Trinder-Typ als auch bei Systemen mit MBTH bzw. MBTH-S sowie Anilin-Derivaten als Farbkupplungspartnern) vermeiden oder wenigstens bis auf ein analytisch nicht mehr ins Gewicht fallendes Ausmaß verringern.

Die folgenden Beispiele erläutern die Erfindung in Verbindung mit der beigefügten Zeichnung. In dieser stellen dar:

Fig. 1 eine graphische Darstellung, in der die SOD-Aktivität gegen die $H_2O_2$-Nachweisempfindlichkeit für die Beispiele 1 bis 3 aufgetragen ist,

Fig. 2 eine graphische Darstellung der Bezugskurven gemäß Beispiel 4,

Fig. 3 eine graphische Darstellung analog Fig. 1 für Beispiel 5.

## Beispiel 1

Verbesserung des $H_2O_2$-Nachweises bei der vollenzymatischen Creatinin-Bestimmung

a) Farbreagenz:

| | |
|---|---|
| TES·KOH (pH 7,9) | 100 mmol/l |
| NaCl | 150 mmol/l |
| 4-Aminoantipyrin | 0,75 mmol/l |
| 2,4,6-Tribrom-3-hydroxybenzoesäure | 8,6 mmol/l |
| Kaliumferrocyanid | 10 µmol/l |
| EDTA | 50 µmol/l |
| Creatininase E.C.3.5.2.10 | 20 U/l |
| Creatinase E.C.3.5.3.3 | 10 U/ml |
| Sarcosin-Oxidase E.C.1.5.3.1 | 6,5 U/ml |
| Peroxidase E.C.1.11.1.7 | 2 U/ml |
| Superoxiddismutase | 0 bis 150 µg/ml ($\hat{=}$ 0 - 600 U/ml) |

b) Testansatz: T = 25°C, $\lambda$ = 546 nm, d = 1,0 cm

|  | Probenwert (P) | Reagenzleerwert (L) |
|---|---|---|
| Farbreagenz | 2,00 ml | 2,00 ml |
| Probe *) | 0,10 ml | - |
| dest. H$_2$O$_2$ | - | 0,10 ml |

20 Minuten inkubieren, dann Extinktion von P gegen L messen. $\Delta E = E_P - E_L$.

*) Als Probenmaterial wurde Preciset® Creatinin, Boehringer Mannheim GmbH, Kat.-Nr. 125 547, eingesetzt. Zur von möglichen Achsenabschnittseffekten unabhängigen Bestimmung der mit 1 mg/dl Creatininkonzentration in der Probe erzielten Extinktionsdifferenz wurden alle Standards als Probe eingesetzt (1 bis 6 mg/dl) und die Steigung der Korrelationsgeraden $\Delta E$ (y-Achse) gegen Creatinin, mg/dl Probe (x-Achse) als Maß für $\Delta E$/mg/dl Creatinin verwendet.

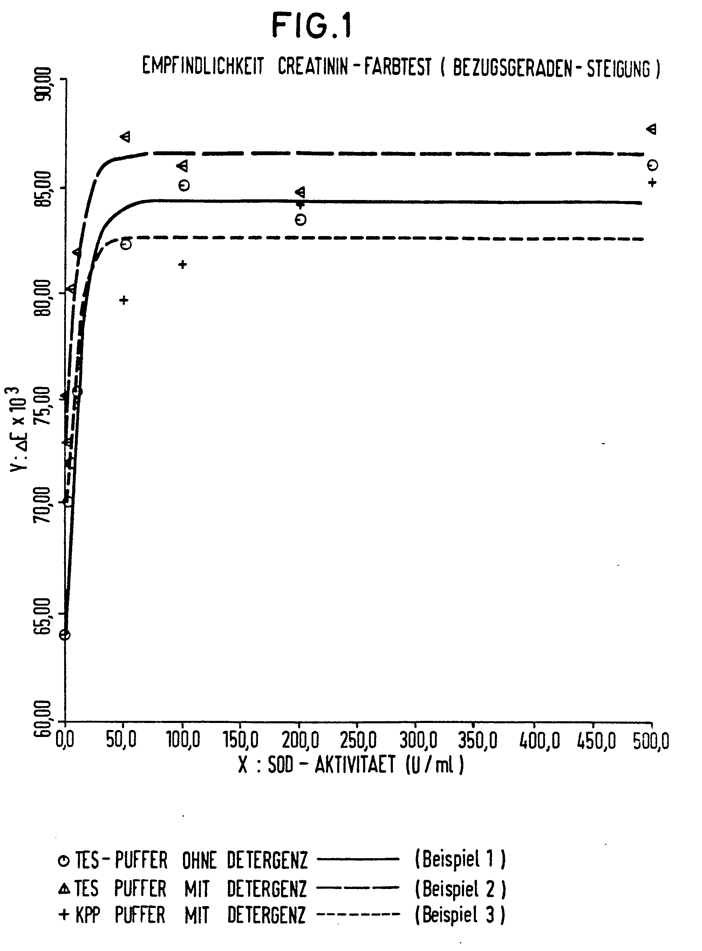

Die Abhängigkeit der Bezugsgeraden-Steigung von der im Farbreagenz enthaltenen Superoxiddismutase-Aktivität (0, 2, 5, 10, 50, 100, 200 und 500 U/ml) zeigt Fig. 1 der Zeichnung (durchzogene Linie). Wie zu ersehen ist, wird gegenüber dem Reagenz ohne Superoxiddismutase ab ca. 50 U/ml Superoxiddismutase-Konzentration im Reagenz ein Plateau mit einer ca. 31 %-igen Empfindlichkeitssteigerung des H$_2$O$_2$-Nachweises erzielt; eine Erhöhung der Superoxiddismutase-Konzentration über 150 µg/ml hinaus ist zwar ohne weiteres möglich, ergibt aber keine Verbesserung in bezug auf die maximal erzielbare Empfindlichkeitssteigerung und ist daher aus wirtschaftlichen Gesichtspunkten nicht bevorzugt. Der negative y-Achsenabschnitt (Bezugsgerade) mit dem Reagenz ohne Superoxiddismutase-Zusatz betrug 0,015 Extinktionseinheiten, bei den Reagenzien mit Superoxiddismutase-Aktivitäten von 50 bis 500 U/ml dagegen lediglich 0,002 Extinktionseinheiten.

Die Ergebnisse ändern sich auch nicht, wenn statt 2,4,6-Tribrom-β-hydroxybenzoesäure die analoge Trijod-Verbindung als Farbkuppler eingesetzt wird.

Wird im obengenannten Farbreagenz Creatininase allein bzw. sowohl Creatininase als auch Creatinase weggelassen, so ist auch die Bestimmung von Creatin bzw. Sarcosin möglich, ohne daß der erfindungsgemäße Effekt des Superoxiddismutase-Zusatzes verloren geht.

**Beispiel 2**

Verbesserung des H$_2$O$_2$-Nachweises bei der enzymatischen Creatinin-Bestimmung

a) Farbreagenz: wie Reagenz a) aus Beispiel 1. Zusätzlich wurden als Aufklarungsmittel 0,25 % n-Decanol-Polyglycolether (Lutensol® ON 50), 5 mmol/l Na-Cholat sowie 2 U/ml Lipase (aus Candida cylindracea) hinzugefügt.

b) Testansatz: Testansatz und Auswertung der Messung erfolgten entsprechend Beispiel 1, Punkt b). Die Meßergebnisse sind in Fig. 1 (gestrichelte Linie (--), Meßpunkte als Dreiecke) wiedergegeben.

**Beispiel 3**

Verbesserung des H$_2$O$_2$-Nachweises bei der enzymatischen Creatinin-Bestimmung

a) Farbreagenz: wie Reagenz a) aus Beispiel 2. Statt des TES-Puffers wurde Kalium-Phosphat-Puffer (pH 7,8), 150 mmol/l, verwendet und der NaCl-Zusatz weggelassen.

b) Testansatz: Testansatz und Auswertung der Messung erfolgten gemäß Beispiel 1, Punkt b). Die Ergebnisse der Messung sind in Fig. 1 (gestrichelte Linie (--), Meßpunkte als Kreuze) wiedergegeben.

**Beispiel 4**

Verbesserung des H$_2$O$_2$-Nachweises bei der enzymatischen Creatinin-Bestimmung

a) Farbreagenz: wie Reagenz a) aus Beispiel 3. Statt Tribromhydroxybenzoesäure wurde jedoch N-Ethyl-N-β-sulfoethyl-m-toluidin (8,6 mmol/l) als Farbkuppler für 4-Aminoantipyrin eingesetzt. Die Superoxiddismutase-Aktivitäten im Reagenz betrugen 0 bzw. 200 U/ml.

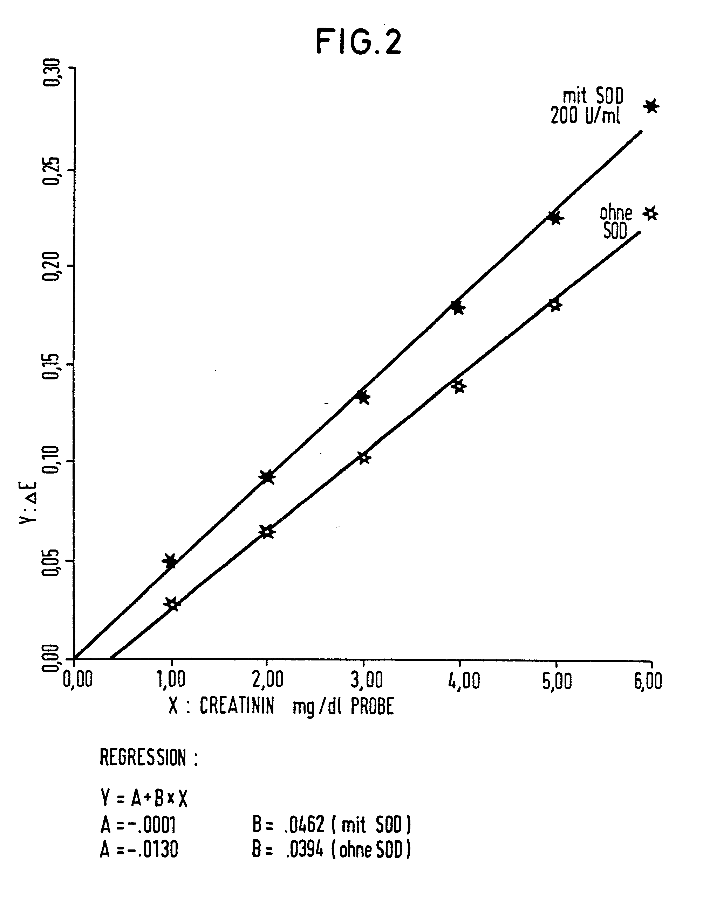

b) Testansatz: erfolgte gemäß Beispiel 1, Punkt b), nur daß nicht bei 546, sonddrn bei 578 nm gemessen wurde. Die Ergebnisse (Bezugskurven) sind in Fig. 2 wiedergegeben. Die Empfindlichkeit dieses Farbindikatorsystems ist zwar niedriger als diejenige des Systems der Beispiele 1 bis 3, doch ist auch hier ersichtlich, daß der Zusatz von Superoxiddismutase sowohl eine deutliche Steigerung der Empfindlichkeit des $H_2O_2$-Nachweises bewirkt (ca. 17,3 %) als auch den mit dem Reagenz ohne Superoxiddismutase-Zusatz gemessenen y-Achsenabschnitt der Korrelationsgeraden (-0,013 Extinktionseinheiten) praktisch völlig zum Verschwinden bringt.

**Beispiel 5**

Verbesserung des $H_2O_2$-Nachweises bei der enzymatischen Harnsäure-Bestimmung

a) Farbreagenz:

| | |
|---|---|
| Kaliumphosphat (pH 8,0) | 100 mmol/l |
| 2,4,6-Tribrom-3-hydroxybenzoesäure | 7,5 mmol/l |
| 4-Aminoantipyrin | 0,2 mmol/l |
| Kaliumferrocyanid | 50 µmol/l |
| EDTA | 1 mmol/l |
| n-Decanolpolyglycolether (Lutensol® ON 50) | 5 g/l |
| Na-Cholat | 7 mmol/l |
| Uricase E.C.1.7.3.3 | 1 U/ml |
| Peroxidase E.C.1.11.1.7 | 3 U/ml |
| Lipase (aus C. cylindracea) E.C.3.1.1.3 | 2 U/ml |
| Superoxiddismutase | 0 bis 500 U/ml |

b) Testansatz: T = 25°C, $\lambda$ = 546 nm, d = 1,0 cm

| | Probenwert (P) | Reagenzleerwert (L) |
|---|---|---|
| Farbreagenz | 2,00 ml | 2,00 ml |
| Probe *) | 0,05 ml | - |
| dest. $H_2O$ | - | 0,05 ml |

10 Minuten inkubieren, dann Extinktion von P gegen L messen. $\Delta E = E_P - E_L$.

*) Als Probenmaterial wurde Preciset® Harnsäure, Boehringer Mannheim GmbH, Kat.-Nr. 125 628, eingesetzt. Zur Bestimmung der mit 1 mg/dl Harnsäure erzielten Extinktionsänderung wurde analog Beispielen 1 bis 3 mit den verschiedenen Harnsäure-Standards (2 bis 12 mg/dl) eine Bezugskurve aufgestellt und deren Steigung als Maß für $\Delta E$/1 mg/dl Harnsäure verwendet.

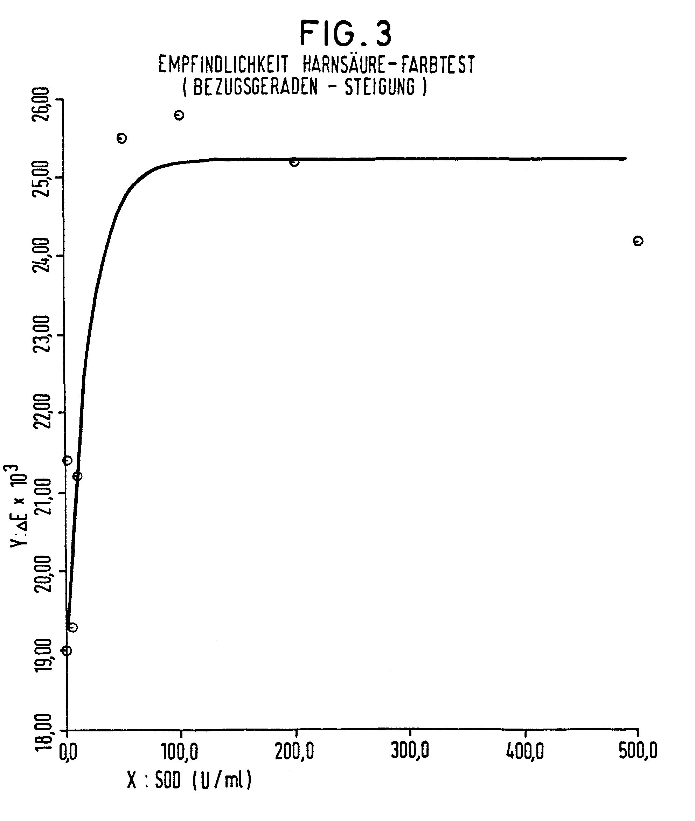

Die Abhängigkeit dieser Steigung ($\Delta E$/1 mg/dl Harnsäure) von der im Farbreagenz eingesetzten Superoxiddismutase-Menge (0, 2, 5, 10, 50, 100, 200 und 500 U/ml) zeigt Fig. 3.

Wie auch in den Beispielen 1 bis 4 kann im Harnsäure-Farbreagenz Tribromhydroxybenzoesäure durch die analoge Trijod-Verbindung oder ein Anilin wie N-Ethyl-N-β-sulfoethyl-m-toluidin als Farbkuppler für 4-Aminoantipyrin ersetzt werden, ohne daß die Verbesserung des $H_2O_2$-Nachweises durch den Superoxiddismutase-Zusatz verloren geht.

**Beispiel 6**

Verbesserung des $H_2O_2$-Nachweises bei der enzymatischen Oxalsäure-Bestimmung mit Hilfe der Oxalat-Oxidase, E.C. 1.2.3.4, sowie der Kombination Peroxidase/MBTH-S/N,N-Dimethylanilin als Farbindikatorsystem

a) Reagenzien

1. Bernsteinsäure-Puffer (0,5 mmol/l; pH = 3,8): 6,75 g Bernsteinsäure-Dinatriumsalz, Hexahydrat, in 400 ml destilliertem Wasser lösen, mit 2 N HCl auf pH 3,8 (25°C) einstellen und auf 500 ml auffüllen.

2. MBTH-S

3. N,N-Dimethylanilin (0,21 mol/l): 100 µl Dimethylanilin in 3,9 ml 0,5 N HCl auflösen.

3. Peroxidase: 1 mg ( = ca. 100 U) in 1 ml destilliertem Wasser lösen.

4. Oxalat-Oxidase: 3,33 mg eines Enzyms mit 0,3 U/mg in 1 ml destilliertem Wasser auflösen.

5. Superoxiddismutase aus Rindererythrozyten (Reinenzym):

12,5 mg in 1 ml destilliertem Wasser lösen und daraus Verdünnungen zu 0,125, 0,250, 2,50 sowie 5,00 mg Enzym pro 1 ml destilliertem Wasser herstellen.

6. Oxalat-Lösungen:

Oxalsäure in Wasser zu Konzentrationen von 1, 2, 3, 4, 5 und 6 mg/dl lösen (bezogen auf 100 % freie Säure).

b) Farb-Grundreagenz

In 100 ml Bernsteinsäurepuffer a)1. 2,3 mg MBTH-S lösen, dann 40 µl Dimethylanilinlösung a)3. zufügen und gut mischen. Zu je 10 ml dieses Gemisches 0,10 ml $H_2O$ oder 0,10 ml der Superpoiddismutase-Lösungen a)5. zufügen (Endkonzentrationen 1,25, 2,50, 25,0, 50,0 und 125,0 µg Superoxiddismutase/ml Grundreagenz) und mischen.

c) Testansatz: T = 37°C, $\lambda$ = 578 nm, d = 1,0 cm

| | Probenwert (P) | Reagenzleerwert (RL) * |
|---|---|---|
| Farb-Grundreagenz | 1,00 ml | 1,00 ml |
| Peroxidase-Lösung | 0,01 ml | 0,01 ml |
| Oxalat-Oxidase-Lösung | 0,10 ml | 0,10 ml |
| Probe (Oxalsäurelösungen) | 0,10 ml | - |
| Wasser | - | 0,10 ml |

20 Minuten inkubieren, dann Extinktionen von P gegen RL messen.

$\Delta E = \Delta E_P - \Delta R_{RL}$

*Pro Meßreihe braucht nur ein Reagenzleerwert mitgeführt werden.

Die Auswertung der mit den mit verschiedenen Mengen an Superoxiddismutase aufgestockten Farbgrundreagenzien erzielten Eichkurven ergab einen deutlichen positiven Einfluß der Superoxiddismutase auf die Beseitigung des y-Achsenabschnitts (y, Extinktionseinheiten, x, Oxalsäure-Probenkonzentration):

**Tabelle I**

Y-Achsenabschnitt der mit den verschieden konzentrierten Oxalsäureproben bei unterschiedlichen Superoxiddismutase-Konzentrationen im Grundreagenz ermittelten Eichgeraden:

| Superoxiddismutase-Konzentration im Farhgrundreagenz (µg/ml) | 0,00 | 1,25 | 2,50 | 25,0 | 50,0 | 125,0 |
|---|---|---|---|---|---|---|
| Y-Achsenabschnitt der Korrelations-geraden (mE) | -29 | -13 | -12 | -5 | -1 | 0 |

**Patentansprüche**

1. Verfahren zur Verbesserung der colorimetrischen Bestimmung von $H_2O_2$, welches durch eine $H_2O_2$-liefernde Oxidase gebildet wird durch Zusatz eines chromogenen Systems und Messung des gebildeten Farbstoffs,
dadurch gekennzeichnet,
daß man als $H_2O_2$-liefernde Oxidase eine nicht $O_2^-$-Radikale bildende Oxidase verwendet und der Reagenzlösung Superoxiddismutase E.C.1.15.1.1 zusetzt.

2. Verfahren nach Anspruch 1,
dadurch gekennzeichnet,
daß man 10 bis 150 µg Superoxiddismutase/ml Reagenzlösung zusetzt.

3. Verfahren nach Anspruch 2,
dadurch gekennzeichnet,
daß man 25 bis 75 µg Superoxiddismutase/ml Reagenzlösung zusetzt.

4. Verfahren nach einem der vorhergehenden Ansprüche,
dadurch gekennzeichnet,
daß man Cu-, Zn-Superoxiddismutase verwendet.

5. Verfahren nach einem der vorhergehenden Ansprüche,
dadurch gekennzeichnet,
daß man als chromogenes System ein solches vom Trindertyp verwendet, welches Peroxidase, Phenol oder ein Phenol- oder Anilinderivat und 4-Aminoantipyrin enthält.

6. Verfahren nach den Ansprüchen 1 bis 4,

dadurch gekennzeichnet,

daß man ein chromogenes System verwendet, das aus Peroxidase, einem Anilinderivat und MBTH bzw. MBTH-S besteht.

7. Verfahren nach Anspruch 5,

dadurch gekennzeichnet,

daß man als Phenolderivat 2,4,6-Trijod-3-hydroxybenzoesäure oder die analoge Tribrom-Verbindung verwendet.

8. Verfahren nach Anspruch 5,

dadurch gekennzeichnet,

daß man als Anilinderivat N-Ethyl-N-β-sulfoethyl-m-toluidin oder N-Ethyl-N-β-hydroxyethyl-m-toluidin verwendet.

9. Verfahren nach Anspruch 6,

dadurch gekennzeichnet,

daß man als Anilinderivat N,N-Dimethylanilin, N,N-Dimethylaminobenzoesäure, N-Ethyl-N-β-sulfoethyl-m-toluidin oder N-Ethyl-N-β-hydroxyethyl-m-toluidin verwendet.

10. Verfahren nach einem der vorhergehenden Ansprüche,

dadurch gekennzeichnet,

daß man als $H_2O_2$-liefernde Oxidase Sarcosinoxidase, Uricase oder Oxalatoxidase verwendet.

11. Reagenz zur verbesserten colorimetrischen Bestimmung von $H_2O_2$, enthaltend eine $H_2O_2$-liefernde Oxidase, ein chromogenes System, Puffer und gegebenenfalls Hilfsenzyme,

dadurch gekennzeichnet,

daß es als $H_2O_2$-liefernde Oxidase eine nicht $O_2^-$-Radikale bildende Oxidase enthält und zusätzlich Superoxiddismutase enthält.

12. Reagenz nach Anspruch 11,

dadurch gekennzeichnet,

daß es Cu-, Zn- Superoxiddismutase enthält.

13. Reagenz nach Anspruch 11 oder 12,

dadurch gekennzeichnet,

daß das chromogene System vom Trindertyp ist und Peroxidase, Phenol oder ein Phenol- oder Anilinderivat und 4-Aminoantipyrin enthält.

14. Reagenz nach Anspruch 11 oder 12,

dadurch gekennzeichnet,

daß das chromogene System Peroxidase, ein Anilinderivat und MBTH bzw. MBTH-S enthält.

15. Reagenz nach Anspruch 13,

dadurch gekennzeichnet,

daß es als Phenolderivat 2,4,6-Trijod-3-hydroxybenzoesäure oder die analoge Tribromverbindung enthält.

16. Reagenz nach Anspruch 13,

dadurch gekennzeichnet,

daß es als Anilinderivat N-Ethyl-N-β-sulfoethyl-m-toluidin oder N-Ethyl-N-β-hydroxyethyl-m-toluidin enthält.

17. Reagenz nach Anspruch 14,

dadurch gekennzeichnet,

daß es als Anilinderivat N,N-Dimethylanilin, N,N-Dimethylaminobenzoesäure, N-Ethyl-N-β-sulfoethyl-m-toluidin oder N-Ethyl-N-β-hydroxyethyl-m-toluidin enthält.

18. Reagenz nach einem der Ansprüche 11 bis 17,

dadurch gekennzeichnet,

daß es als $H_2O_2$-liefernde Oxidase Sarcosinoxidase, Uricase oder Oxalatoxidase enthält.

19. Reagenz nach einem der Ansprüche 11 bis 18,

dadurch gekennzeichnet,

daß es 10 bis 150 µg Superoxiddismutase pro ml enthält.

20. Reagenz nach einem der Ansprüche 11 bis 19, dadurch gekennzeichnet,

daß es zusätzlich eine oder mehrere Substanzen aus der Gruppe Aufklarungsmittel, Konservierungsmittel, Mittel zur Beseitigung der Störungen durch Ascorbinsäure oder Bilirubin und Schwermetallkomplexbildner enthält.

21. Reagenz nach einem der Ansprüche 11 bis 20,

dadurch gekennzeichnet,

daß es auf einem festen inerten, gegebenenfalls mehrschichtigen Trägermaterial vorliegt.

## Claims

1. Process for the improvement of the colorimetric determination of $H_2O_2$, which is formed by an $H_2O_2$-producing oxidase, by addition of a chromogenic system and measurement of the coloured material formed, characterised in that, as $H_2O_2$-producing oxidase, one uses a non-$O_2^-$ radical-forming oxidase and adds superoxide dismutase, E.C. 1.15.1.1, to the reagent solution.

2. Process according to claim 1, characterised in that one adds 10 to 150 µg of superoxide dismutase/ml reagent solution.

3. Process according to claim 2, characterised in that one adds 25 to 75 µg of superoxide dismutase/ml reagent solution.

4. Process according to one of the preceding claims, characterised in that one uses Cu, Zn-superoxide dismutase.

5. Process according to one of the preceding claims, characterised in that, as chromogenic system, one uses one of the Trinder type which contains peroxidase, phenol or a phenol or aniline derivative and 4-aminoantipyrine.

6. Process according to claims 1 to 4, characterised in that one uses a chromogenic system which consists of peroxidase, an aniline derivative and MBTH or MBTH-S.

7. Process according to claim 5, characterised in that, as phenol derivative, one uses 2,4,6-triiodo-3-hydroxybenzoic acid or the analogous tribromo compound.

8. Process according to claim 5, characterised in that, as aniline derivative, one uses N-ethyl-N-β-sulphoethyl-$\underline{m}$-toluidine or N-ethyl-N-β-hydroxyethyl-$\underline{m}$-toluidine.

9. Process according to claim 6, characterised in that, as aniline derivative, one uses N,N-dimethylaniline, N,N-dimethylaminobensoic acid, N-ethyl-N-β-sulphoethyl-$\underline{m}$-toluidine or N-ethyl-N-β-hydroxyethyl-$\underline{m}$-toluidine.

10. Process according to one of the preceding claims, characterised in that, as $H_2O_2$-producing oxidase, one uses sarcosine oxidase, uricase or oxalate oxidase.

11. Reagent for the improved colorimetric determination of $H_2O_2$ containing an $H_2O_2$-producing oxidase, a chromogenic system, buffer and optionally adjuvant enzymes, characterised in that it contains a non-$O_2^-$ radical-forming oxidase as $H_2O_2$-producing oxidase and additionally superoxide dismutase.

12. Reagent according to claim 11, characterised in that it contains Cu, Zn-superoxide dismutase.

13. Reagent according to claim 11 or 12, characterised in that the chromogenic system is of the Trinder type and contains peroxidase, phenol or a phenol or aniline derivative and 4-aminoantipyrine.

14. Reagent according to claim 11 or 12, characterised in that the chromogenic system contains peroxidase, an aniline derivative and MBTH or MBTH-S.

15. Reagent according to claim 13, characterised in that, as phenol derivative, it contains 2,4,6-triiodo-3-hydroxybenzoic acid or the analogous tribromo compound.

16. Reagent according to claim 13, characterised in that, as aniline derivative, it contains N-ethyl-N-β-sulphoethyl-$\underline{m}$-toluidine or N-ethyl-N-β-hydroxyethyl-$\underline{m}$-toluidine.

17. Reagent according to claim 14, characterised in that, as aniline derivative, it contains N,N-dimethylaniline, N,N-dimethylaminobenzoic acid, N-ethyl-N-β-sulphoethyl-$\underline{m}$-toluidine or N-ethyl-N-β-hydroxyethyl-$\underline{m}$-toluidine.

18. Reagent according to one of claims 11 to 17, characterised in that, as $H_2O_2$-producing oxidase, it contains sarcosine oxidase, uricase or oxalate oxidase.

19. Reagent according to one of claims 11 to 18, characterised in that it contains 10 to 150 µg superoxide dismutase per ml.

20. Reagent according to one of claims 11 to 19, characterised in that it additionally contains one or more substances of the group clarification agents, preserving agents, agents for the removal of disturbances due to ascorbic acid or bilirubin and heavy metal complex formers.

21. Reagent according to one of claims 11 to 20, characterised in that it is present on a solid inert, optionally multi-layer carrier material.


## Revendications

1. Procédé pour l'amélioration du dosage colorimétrique de $H_2O_2$ qui est produit par une oxydase génératrice d'$H_2O_2$, par addition d'un systeme chromogène et mesure du colorant formé, caractérisé en ce qu'on emploie comme oxydase génératrice d'$H_2O_2$ une oxydase ne formant pas de radicaux $O_2^-$ et ajoute à la solution de réactif de la superoxyde dismutase E.C.1.15.1.1.

2. Procédé selon la revendication 1, caractérisé en ce qu'on ajoute 10 à 150 µg de superoxyde dismutase/ml de solution de réactif.

3. Procédé selon la revendication 2, caractérisé en ce qu'on ajoute 25 à 75 µg de superoxyde dismutase/ml de solution de réactif.

4. Procédé selon l'une des revendications précédentes, caractérisé en ce qu'on utilise de la superoxyde dismutase-Cu, Zn.

5. Procédé selon l'une des revendications précédentes, caractérisé en ce qu'on utilise comme système chromogene un système de type Trinder qui contient une peroxydase, du phénol ou un dérivé du phénol ou de l'aniline et de la 4-aminoantipyrine.

6. Procédé selon les revendications 1 à 4, caractérisé en ce qu'on utilise un système chromogène constitué par une peroxydase, un dérivé de l'aniline et de la MBTH, respectivement de la MBTH-S.

7. Procédé selon la revendication 5, caractérisé en ce qu'on utilise comme dérivé du phénol l'acide 2,4,6-triiodo-3-hydroxybenzoïque ou le composé tribromo analoque.

8. Procédé selon la revendication 5, caractérisé en ce qu'on utilise comme dérivé de l'aniline la N-éthyl-N-$\beta$-sulfoéthyle-m-toluidine ou la N-éthyl-N-$\beta$-hydroxyéthyl-m-toluidine.

9. Procédé selon la revendication 6, caractérisé en ce qu'on utilise comme dérivé de l'aniline la N,N-diméthylaniline, l'acide N,N-diméthylaminobenzoïque, la N-éthyl-N-$\beta$-sulfoéthyl-m-toluidine ou la N-étnyl-N-$\beta$-hydroxyéthyl-m-toluidine.

10. Procédé selon l'une des revendications précédentes, caractérisé en ce qu'on utilise comme oxydase génératrice d'$H_2O_2$ la sarcosine oxydase, l'uricase ou l'oxalate oxydase.

11. Réactif pour le dosage colorimétrique amelioré de $H_2O_2$, contenant une oxydase génératrice d'$H_2O_2$, un système chromogène, un tampon et, le cas échéant, des enzymes auxiliaires, caractérisé en ce qu'il contient comme oxydase génératrice d'$H_2O_2$ une oxydase ne formant pas de radicaux $O_2^{\cdot-}$ et en outre de la superoxyde dismutase.

12. Réactif selon la revendication 11, caractérisé en ce qu'il contient de la superoxyde dismutase - Cu, Zn.

13. Réactif selon la revendication 11 ou 12, caractérisé en ce que le système chromogène est du type Trinder et contient une peroxydase, du phénol ou un dérivé du phénol ou de l'aniline et de la 4-aminoantipyrine.

14. Réactif selon la revendication 11 ou 12, caractérisé en ce que le système chromogène contient une peroxydase, un dérivé de l'aniline et de la MBTH, respectivement de la MBTH-S.

15. Réactif selon la revendication 13, caractérisé en ce qu'il contient comme dérivé du phénol l'acide 2,4,6-triiodo-3-hydroxybenzoïque ou le dérivé tribromo analogue.

16. Réactif selon la revendication 13, caractérisé en ce qu'il contient comme dérivé de l'aniline la N-éthyl-N-$\beta$-sulfoéthyl-m-toluidine ou la N-éthyl-N-$\beta$-hydroxyéthyl-m-toluidine.

17. Réactif selon la revendication 14, caractérisé en ce qu'il contient comme dérivé de l'aniline la N,N-diméthylaniline, l'acide N,N-diméthylaminobenzoïque, la N-éthyl-N-$\beta$-sulfoéthyl-m-toluidine ou la N-éthyl-$\beta$-hydroxyéthyl-m-toluidine.

18. Réactif selon l'une des revendications 11 à 17, caractérisé en ce qu'il contient comme oxydase génératrice d'$H_2O_2$ la sarcosine oxydase, l'uricase ou l'oxalate oxydase.

19. Réactif selon l'une des revendications 11 à 18, caractérisé en ce qu'il contient 10 à 150 µg de superoxyde dismutase par ml.

20. Réactif selon l'une des revendications 11 à 19, caractérisé en ce qu'il contient en outre une ou plusieurs substances du groupe des clarifiants, des agents de conservation et des agents pour éviter les interférences dues à l'acide ascorbique ou la bilirubine et des complexants des métaux lourds.

21. Réactif selon l'une des revendications 11 à 20, caractérisé en ce qu'il se trouve sur un support solide inerte, éventuellement multicouche.

**EP 0 186 134 B1**

# FIG.1

EMPFINDLICHKEIT CREATININ – FARBTEST ( BEZUGSGERADEN – STEIGUNG )

Y : $\Delta E \times 10^3$

X : SOD – AKTIVITAET (U / ml )

⊙ TES – PUFFER OHNE DETERGENZ ———————— (Beispiel 1 )

△ TES PUFFER MIT DETERGENZ ——— — ——— (Beispiel 2 )

+ KPP PUFFER MIT DETERGENZ – – – – – – – (Beispiel 3 )

1

# FIG.2

REGRESSION :

$Y = A + B \times X$

A = -.0001      B = .0462 ( mit SOD )
A = -.0130      B = .0394 ( ohne SOD )

**FIG. 3**

EMPFINDLICHKEIT HARNSÄURE–FARBTEST
( BEZUGSGERADEN – STEIGUNG )